# EUROPEAN PATENT APPLICATION

(11) **EP 0 791 360 A2**
(43) Date of publication of application: **27.08.1997**
(21) Application number: 97102619.0
(22) Date of filing: 19.02.1997
(51) Int. Cl.: A61K 39/395, C07K 16/24, C12N 5/12

(54) **Treatment of septic shock with anti-TNF antibodies**

(30) Priority: 29.02.1996 US 609022
(71) Applicant: Bayer Corporation, Pittsburgh, PA 15219-2502 (US)
(72) Inventor: Jesmok, Gary, Madison, Connecticut 06443 (US); Gundel, Robert, Walnut Creek, California 94595 (US)
(74) Representative: Burkert, Frank

(57) **Abstract**

A method of treating a mammal in an established state of septic shock, the method comprising administering a clinically effective dose of anti-TNF monoclonal antibodies to the mammal, said antibodies being administered under conditions sufficient to alleviate the state of shock and reduce the likelihood of mortality.

## Description

### BACKGROUND OF THE INVENTION

Field This invention generally relates to treating septic shock in mammals and more specifically relates to using a monoclonal antibody specific for tumor necrosis factor to treat a mammal in a clinical state of shock.

Background Septic shock due to infections by gram-negative and gram-positive bacteria is a major clinical problem associated with a high mortality of between 40-60% (1, 2). Septic shock is a complex medical syndrome characterized by hypotension, vascular leakage, and multi-organ failure and is thought to develop from an errant systemic inflammatory response (3, 4). Tumor Necrosis Factor (TNF) has been demonstrated to play a prominent role in the systemic inflammatory reaction which can develop into septic shock. TNF is measured in the plasma of patients who are in septic shock, and TNF infusion mimics many characteristics of septic shock. Furthermore, neutralization of TNF has been demonstrated to protect animals against the lethal effects of intravenous endotoxin or live bacteria challenge (5, 6).

Many studies have demonstrated that TNF blockade can prevent endotoxin induced septic shock (11, 12). All of the protection studies have either pretreated the animals or given the antibody very early alter the intravenous endotoxin or live bacterial challenge (7-12). In these latter studies the animals were not in a clinically defined state of shock before the antibody was administered. Several ongoing clinical trials are investigating the therapeutic utility of TNF blockade in the treatment of septic shock (13). One of the clinical trials, the NORASEPT II trial, is designed to treat established shock. Although previous investigators have demonstrated benefit with pretreatment regimens, to our knowledge no one has demonstrated the benefit of TNF blockade in the treatment of established septic shock. Septic shock in these studies is defined as per guidelines published by the Society of Critical Care Medicine as sepsis associated with hypotension and evidence of tissue hypoperfusion (22). Sepsis has been defined by the same group as 2 or more of the following; fever, tachycardia, tachypnea, leukocytosis or leukopenia. We sought to induce sepsis followed quickly by septic shock in pigs using these agreed upon criteria i.e., sepsis with hypotension and evidence of hypoperfusion and evaluate the efficacy of TNF blockade with monoclonal antibody. Numerous models of septic shock are available (14, 15). Many of these entail the infusion of large amounts of endotoxin or live bacteria, although endotoxin is preferred as it is difficult to control the dosing with live bacteria. The endotoxemic pig is an excellent, well characterized model of septic shock (16-18). The pig is responsive to low dose of LPS, similar to humans. Systemic hemodynamics can be measured in the pig to allow intervention at the appropriate time when shock is evident. Furthermore, the swine model of endotoxemia is well characterized and is thought to mimic the cardiopulmonary response of humans in septic shock (19, 20). In addition, an anti-TNF monoclonal antibody (denoted BAY 1351, ATCC HB 9736, deposited June 8, 1988) has been demonstrated to neutralize swine TNF (9). In order to make septic shock in the swine have very similar characteristics to septic shock in humans we used a thromboxane A₂ receptor antagonist (BAY U 3405). Thromboxane A₂ blockade with a receptor antagonist (BAY U 3405) was utilized in this septic shock model to prevent severe pulmonary hypertension and early (<60 minutes) mortality. A paper describing this model has been published (21). BAY U 3405, (3R)-3-(4-fluorophenyl-sulfonamido)-1,2,3,4, tetrahydro-9-carbazolepropanoic acid), is a competitive and selective thromboxanc A₂ antagonist.

Septic shock as defined per accepted clinical criteria (see above) was unresponsive to standard therapy which included fluids and vasopressors. Since all of the previous experimental work with anti-TNF antibodies has looked at prevention of the development of shock, we thought it appropriate to experimentally examine the therapeutic benefit of TNF Mab in animals with established septic shock. Therefore, we sought to examine the effect of TNF blockade after the induction of shock by inducing septic shock in swine and then treating the septic shock condition with TNF Mab (BAY 1351) in a manner somewhat similar to the ongoing NORASEPT II trial. We quite surprisingly discovered that, unlike previous work which showed only prophylaxis, we could treat already established cases of shock in a clinically effective manner.

### SUMMARY OF THE INVENTION

We have now found a method of treating established cases of septic shock in a model mammalian system that is thought to be applicable to humans. The method comprises intravenously administering a clinically effective aqueous solution of anti-TNF monoclonal antibodies (TNF Mab) to a mammal experiencing a clinically established case of septic shock. The TNF Mab is administered in amounts and under conditions which result in reduction in the symptoms of shock and increased survival of the treated mammals versus control mammals.

A preferred dosage is in the range of 1 to 10 mg/kg of mammal weight, given as soon as possible alter the established state of shock is determined. In the examples below, the antibodies were administered at a time interval of 30-60 minutes after the onset of established shock. It is thought that the window of therapeutic opportunity would be wider (e.g. about 1-12 hrs) depending on the severity of shock. That is, in the clinic setting, septic shock probably does not develop as rapidly as it does in our model. However, our model demonstrates for the first time that it is possible to reverse septic shock with TNF Mab treatment. Under less severe conditions such as likely occurs in the critical care setting, one would most likely would have a much longer period of time to intervene. It is thought that the treatment disclosed herein is applicable to a group of mammals including pigs and humans.

### BRIEF DESCRIPTION OF THE FIGURES

**Figures 1A, 1B and 1C** show the effect of treatment of shock with TNF Mab on systemic hemodynamics in endotoxemic swine. Shock was induced with LPS infused at a rate of 10 µg/kg/hr. TNF Mab (10 mg/kg) was administered when shock was extant, or clinically established, i.e. systolic pressure <90 mmHg unresponsive to fluids. Results are presented as mean ± SEM. * Indicates a value that is statistically different from control, p < 0.05.
**Figures 2A and 2B** show the effect of treatment of shock with TNF Mab on cardiac function in endotaxemic swine. Shock induction and intervention as per Figure 1. Results are presented as mean ± SEM. * Indicates a value that is statistically different from control, p < 0.05.
**Figures 3A and 3B** show the effect of treatment of shock with TNF Mab on pulmonary dynamics in endotoxemic swine. Shock induction and intervention as per Figure 1. Results are presented as mean ± SEM. * Indicates a value that is statistically different from control, p < 0.05.
**Figures 4A and 4B** show the hematologic effects of treatment of shock with TNF Mab in endotoxemic swine. Shock induction and intervention as per Figure 1. Results are presented as mean ± SEM. * Indicates a value that is statistically different from control, p < 0.05.
**Figures 5A, 5B and 5C** show the metabolic effects of treatment of shock with TNF Mab in endotoxemic swine. Shock induction and intervention as per Figure 1. Results are presented as mean ± SEM. * Indicates a value that is statistically different from control, p< 0.05.

### SPECIFIC EMBODIMENTS

### Materials and Methods

Domestic pigs (12-20 kgs) were fasted for 12 hrs prior to the induction of anesthesia by IM injection of xylazine (8 mg/kg) and ketamine (15 mg/kg), intubated with a cuffed endotracheal tube, and maintained with 1-2% isoflurane anesthetic (Ohio medical). The animals were prepared for aseptic cannulation of the common carotid and jugular vein with 19 gauge vialon resin catheters (Intercath, Desert Medical Inc., Sandy UT). A 5F Swan Ganz thermodilution catheter (Edwards Critical Care, Santa Ana, CA) was advanced into the pulmonary artery via the jugular vein for monitoring cardiopulmonary function. Pulmonary and arterial pressures were continuously monitored on a Gould 2800 S chart recorder using a P28 ID pressure transducer (Gould Inc., Cupertino, CA). Cardiac output was determined by thermodilution technique via injection of chilled 0.9% NaCl solution. All animals were treated with thromboxane receptor antagonist (BAY U 3405, 1 mg/kg) to prevent early pulmonary hypertension and premature mortality. (The reason for this is explained in a previous publication (21).) Citrated blood samples were taken hourly for determination of metabolic and inflammatory parameters. Blood samples were also collected in EDTA, and a complete hematology profile was obtained using a SERONO-BAKER 9000 Hematology System. Total leukocyte counts were made utilizing a TECHNICON H 1ETM SYSTEM (Bayer Corp., Tarrytown NY). Blood samples were also used for determination of lactate and glucose levels using a YELLOW SPRINGS INSTRUMENT 2700. These measurement were made to insure that the swine was in a state of clinically defined shock as per ACCP/SCCM definitions (22).

Following baseline cardiopulmonary' measurements and blood sampling, endotoxin (055:B5) was infused at a rate of µg/kg/hr. Constant infusion more closely mimics what may occur in the hospital, if the patient has an infection and the bacteria are shedding endotoxin into the circulation. We had demonstrated in previous experiments that this is a LD₁₀₀ dose of endotoxin at 12-24 hrs. Cardiopulmonary measurements were taken every 30 minutes for the first 2 hours and hourly thereafter for 5 hours. This, again, is what might occur in a clinical ICU. When shock was extant, e.g. arterial systolic pressure had declined to below 90 mmHg or had fallen 40 mmHg from baseline and the hypotension was unresponsive to fluid therapy (500-1500 ml of lactated Ringers solution), TNF Mab (BAY 1351, 10 mg/kg) was administered. A non-specific antibody, i.e. IgG, was given in the control group. Other evidence of shock was also present before the TNF Mab was administered. The animal had a spike in body temperature, respiration increased, cardiac output was comprised, heart rate was elevated, stroke volume was depressed, leukocytes and platelet levels were low, lactate levels had increased. Thus, all evidence demonstrated that the animals were in **shock** as per ACCP/SCCM definition. Cardiopulmonary measurement and blood samples were taken over the following 5 hrs.

At 5 hrs the catheters were removed and the cannulated blood vessels were repaired with 6-0 silk suture, the skin was sutured, and the animals were returned to cages for observation. Survival and the development of organ failure was monitored for 3-5 days.

### Material Preparation

TNF Mab (Bay 1351) was produced at Bayer Corporation., Biotechnology Division, Berkeley, California. The murine monoclonal antibody (IgG1) was produced by hybridoma culture and purified from culture harvests by cell separation, polyethylene glycol precipitation, anion exchange and size exclusion chromatography. The final preparation was > 99% pure, and the endotoxin content was < 2.0 pg/mg protein. This antibody, although raised against recombinant human TNF, effectively neutralized swine TNF (9).

Sixty milligrams of BAY U 3405 (Bayer UK) was dissolved in 20 ml of a solution of 1 M NaOH and phosphate buffered saline (PBS) and adjusted to pH 7.0 with 1M HCL. Animals were dosed at 1 mg/kg. A stock solution of 6 mg/ml was used for all experiments.

### Statistical Analysis

Data are represented as means ± SEM. There were 10 animals/group. The chisquare test was used to determine significance where the end point was survival. The differences between groups for cardiopulmonary, metabolic and hematologic variables were tested by one way analysis of variance (ANOVA), and Duncan's multiple comparisons was used to test for significant differences between groups. Probability values below 0.05 were considered statistically significant.

### RESULTS

### Effect of Treatment of Septic Shock with TNF Mab on Systemic Hemodynamics and Cardiac Function

The intravenous infusion of LPS at a rate of 10 µg/kg/hr resulted in the development of shock within 60-90 minutes. Shock was defined as a systolic pressure less than 90 mmHg or a fall in systolic pressure of 40 mmHg.

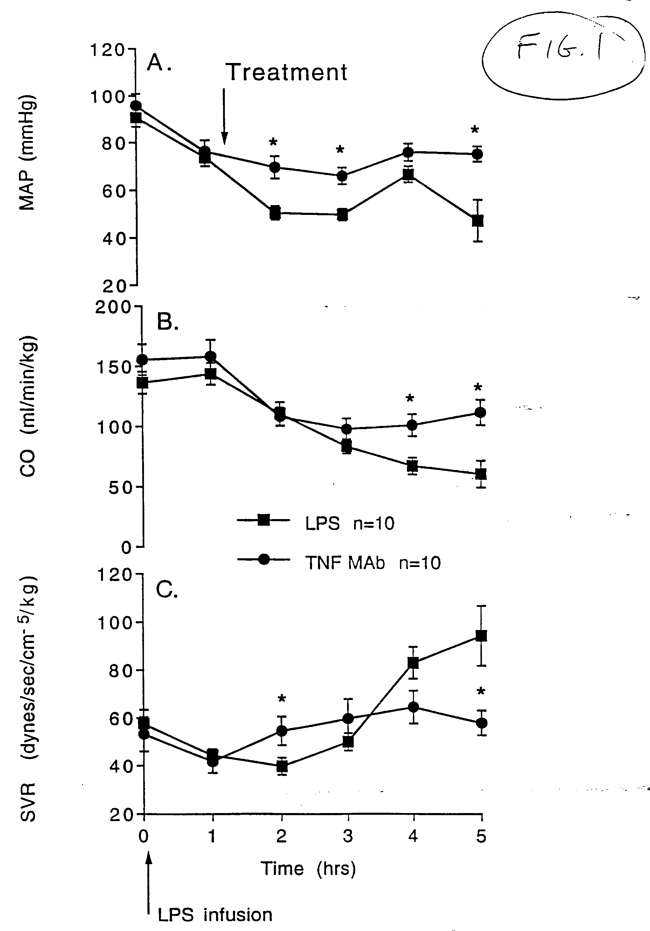

The model employed herein exhibits all the clinical manifestations of septic shock including fever, tachycardia, tachypnea, refractory hypotension, vascular leakage, evidence of tissue hypoperfusion (elevated lactate), organ failure and a high mortality. Furthermore, similar to humans in septic shock, it was unresponsive to fluids (lactated ringers) and vasopressors (dopamine or epinephrine) were unable to protect the animal from mortality. The shock state was characterized by a decrease in mean arterial pressure (MAP), cardiac output (CO) and systemic vascular resistance (SVR, Fig 1). Treatment of shock with TNF Mab (BAY 1351) resulted in a significant elevation of MAP from 90-300 minutes compared to untreated animals. In addition, the decline in CO was arrested by TNF neutralization, and CO was significantly increased at 240 and 300 minutes. The decline in CO induced by LPS is most likely related to decreased venous return, depressed myocardial contractility, and microvascular leakage. The effect of LPS on SVR is biphasic in untreated animals. There is an initial decrease over 1-2 hrs followed by a dramatic increase at 4-5 hrs as CO declines. This latter pronounced increase in SVR in the presence of hypotension was normally followed within 1-3 hours by death and may be related to an agonal surge in the release of catecholamines. TNF neutralization stabilized SVR most likely through the maintenance of CO.

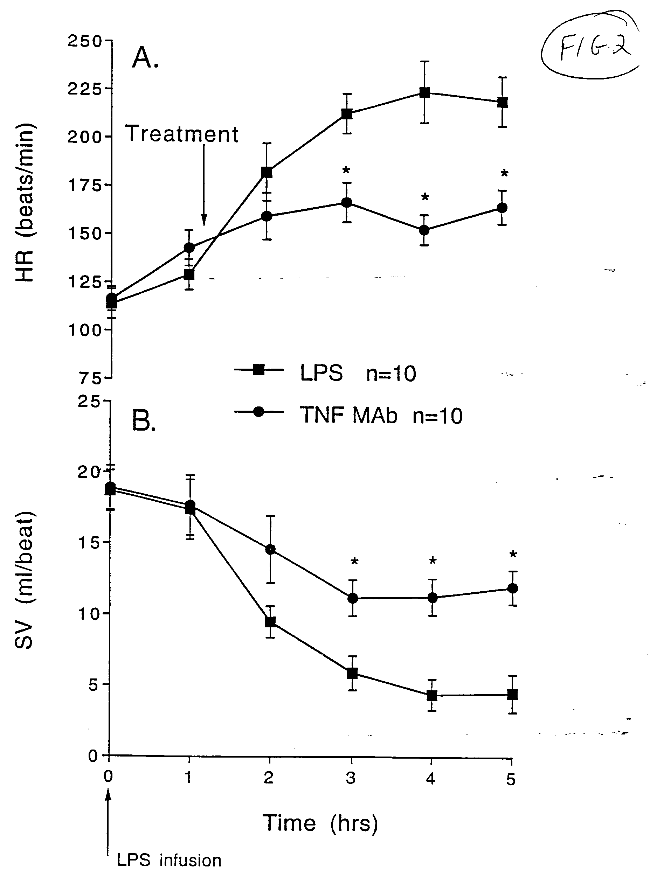

As shock developed and MAP declined, heart rate (HR) inch (Fig 2, A). The increase in. HR is probably related to hypotension-induced activation of baroreflex as well as endogenous release of catecholamines. The increased HR and the decline in CO resulted in a pronounced decrease in stroke volume (SV, Fig 2 B). Treatment of shock with TNF Mab significantly decreased HR and stabilized SV from 3-5 hrs.

### Effect of Treatment of Septic Shock with TNF Mab on Pulmonary Dynamics

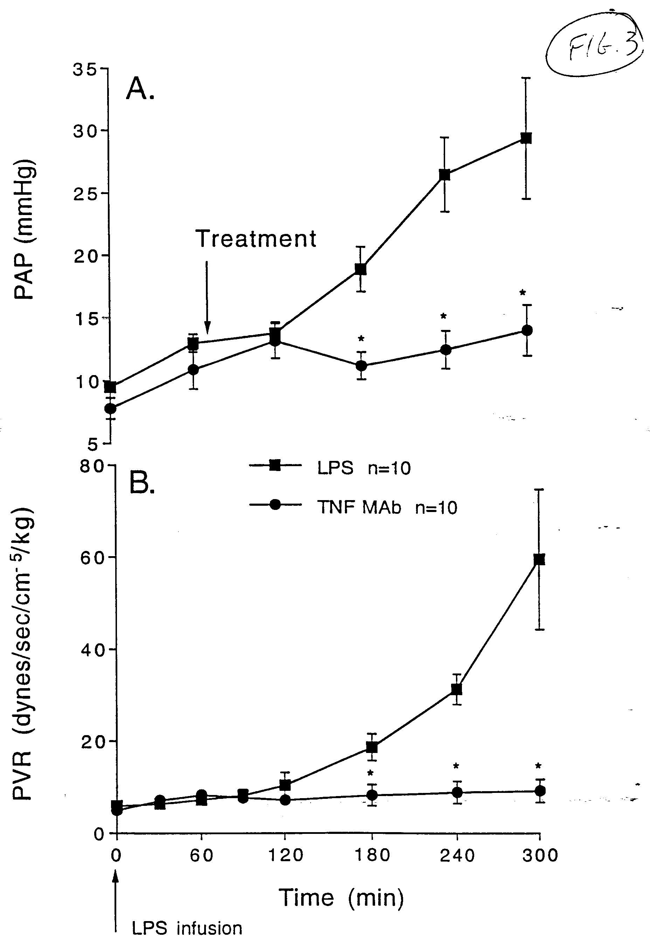

In this model of septic shock, the early (15-30 minutes) pulmonary hypertension which normally develops in swine challenged with endotoxin was blocked with the thromboxane antagonist (BAY U 3405, data not shown). This was done to avoid the acute pulmonary response to LPS which can result in early mortality <60 minutes. Importantly, thromboxane blockade does not effect the latter development of systemic shock (21). Therefore this model still exhibits all the clinical manifestations of septic shock, e.g. refractory hypotension, vascular leakage, organ failure, and a high mortality. As septic shock developed, thee was a second phase response in the lung with pulmonary artery pressure (PAP) and pulmonary vascular resistance (PVR) increasing independent of thromboxane antagonism (Fig 3). This second phase (3-5 hrs) increase in PAP and PVR was blocked by treatment with TNF Mab.

### Effect of Treatment of Septic Shock with TNF Mab on Hematologic Variables

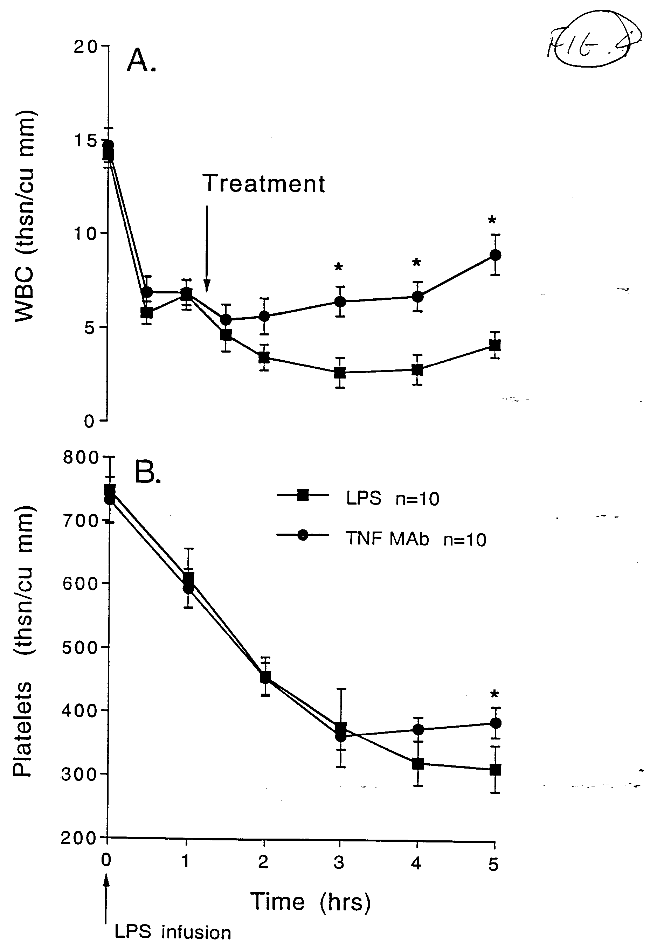

The development of shock in the endotoxemic swine was characterized by a pronounced leukopenia and thrombocytopenia (Fig 4). Treatment of shock with TNF Mab resulted in a significant increase in circulating leukocytes without a noticeable effect on platelet levels except at 300 minutes.

### Effect of Treatment of Septic Shock with TNF Mab on Metabolic Variables

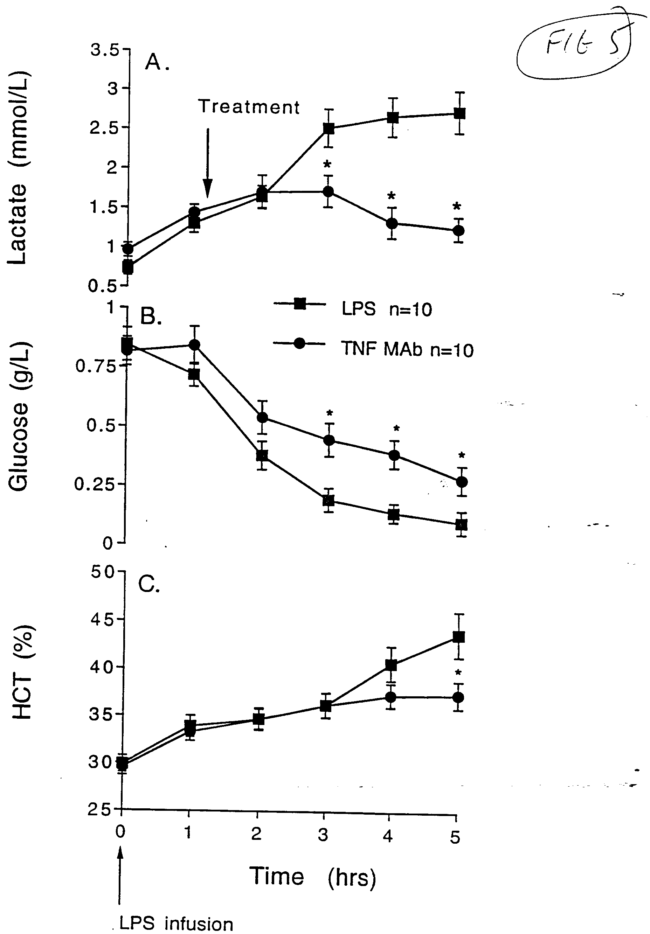

The development of shock was associated with an elevation of blood lactate levels, a decrease in blood glucose, and an increase in hematocrit (HCT) which was very pronounced between 4-5 hrs (Fig 5). The increase in blood lactate is most likely related to decreased organ perfusion and O₂, delivery to peripheral tissues as shock develops. The hypoglycemia may be related to a switch to anaerobic metabolism as O₂ delivery is compromised during shock. The latter increase in HCT appears to be largely due to an increase in microvascular permeability and a loss of plasma volume into the extravascular space, a characteristic of the shock state. Treatment of shock with TNF Mab significantly decreased the rise in blood lactate and increased glucose levels between 3-5 hrs. Furthermore TNF neutralization blocked the increase in HCT between 3-5 hrs.

### Effect of Treatment of Shock with TNF Mab on Mortality

Mortality data is shown in the Table. In the control animals (not treated with TNF Mab) mortality was 100% at 24 hrs with most of the deaths occurring between 5-7 hrs, whereas in the TNF Mab treated group only 1 of 10 animals expired (10%), p<0.01 treated versus non-treated. Animals treated with TNF Mab were alert and began eating soon after recovering from anesthesia (<60 minutes). In striking contrast, animals not treated with TNF never fully recovered following the induction of shock.

**Table**

| Effect of Treatment of Septic Shock with TNF Mab on Mortality | |
|---|---|
| Treatment | Mortality (%) |
| - TNF Mab | 10/10 (100%) |
| + TNF Mab | 1/10 (10%)* |

| | |
|---|---|
| *P< 0.01 TNF Mab versus Control Animals | |

### CONCLUSION

To our knowledge, the experiments described herein demonstrate for the first time the efficacy of an anti-TNF antibody such as TNF Mab (BAY 1351) in the treatment of established septic shock in a clinically relevant experimental animal. All previous work with TNF Mab has used a pretreatment regimen or has given the antibody before demonstrable shock had developed (7-12). The septic shock model we use has been previously described (21), and the pig model is well characterized and is a standard, well accepted model of septic shock (16-20) thought to be applicable to other mammals including humans. The antibody we used is the monoclonal derived against human TNF (BAY 1351), ATCC HB 9736, deposited June 8, 1988 by Chiron Corporation and licensed to Bayer Corporation.

In this model, we have used a number of clinically relevant measurements to demonstrate that the animal was in a state of shock when the antibody was administered. These included hemodynamics (Figs. 1A - 1C), cardiac function (Figs. 2A - 2B), hematology (Figs 4A - 4B) and metabolic (Figs. 5A - 5C) parameters and inability of fluids or standard vasopressors to protect the animal. A similar battery of tests is used in the clinical ICU to assess whether a patient is in shock; therefore, the measurement we have made have clinical applicability. TNF Mab treatment of shock improved all of these measured parameters and, most significantly, promoted permanent survival (Table).

The salient feature of this work is that we have demonstrated for the first time a striking benefit conferred by TNF Mab in established septic shock in an experimental model which is clinically relevant.

The above examples are intended to illustrate the invention and it is thought variations will occur to those skilled in the art. Accordingly, it is intended that the scope of the invention should be limited only by the claims below.

### REFERENCES

1. Parillo, J.E., et al., "Septic Shock in Humans", *Ann. Intern. Med.* **113:** 227-242 (1990).
2. Bone, R., et al., "Toward an Epidemiology and Natural History of SIRS (Systemic Inflammatory Response Syndrome)", *JAMA* **101:**1481-1483 (1992).
3. Bone, R.C., "The Pathogenesis of Sepsis", *Ann. Intern. Med.* **115:** 457-469 (1991).
4. Parrillo, J.E. "Pathogenetic Mechanisms of Septic Shock", *N. Engl. J. Med.* **328:** 1471-1477 (1993).
5. Tracey, K.J., "Tumor Necrosis Factor (Cachectin) in the Biology of Septic Shock Syndrome", *Circ. Shock* **35:** 123-128 (1991).
6. Giroir, B., "Mediators of Septic Shock: New Approaches for Interrupting the Endogenous Inflammatory Cascade", *Crit. Care Med.* **21**: 318-327 (1993).
7. Tracey, K.J., et al., "Anti-Cachetin/TNF Monoclonal Antibodies Prevent Septic Shock During Lethal Bacteremia", *Nature* **330:** 662-664 (1987).
8. Hinshaw, L.B., et al., "Survival of Primates in LD₁₀₀ Septic Shock Following Therapy with Antibody to TNF", *Circ. Shock* **30:** 279-292 (1990).
9. Jesmok, G.J., et al., "Efficacy of Monoclonal Antibody Against Recombinant Tumor Necrosis Factor in E. coli-challenged swine", *Am. J. Path* **141:** 1197-1207 (1992).
10. Walsh, C.J., et al., "Monoclonal Antibody to Tumor Necrosis Factor Alpha Attenuates Cardiopulmonary Dysfunction in Porcine Gram Negative Sepsis", *Arch*. *Surg*. **127:** 138-145 (1992).
11. Fiedler, V.B., et al., "Monoclonal Antibody to Tumor Necrosis Factor Prevents Lethal Endotoxin Sepsis in Adult Rhesus Monkeys", *J. Lab. Clin. Med.* **120:** 574-588 (1992).
12. Emerson, T.E., et al., Efficacy of Monoclonal Antibody Against Tumor Necrosis Factor Alpha in a Low Mortality Endotoxemic Baboon Model", *Cir. Shock* **38:** 775-784 (1992).
13. Abraham, E., et al., "Efficacy and Safety of Monoclonal Antibody to Human Tumor Necrosis Factor ∂ in Patients with Sepsis Syndrome", *JAMA* **273:** 934-941 (1995).
14. Wichterman, K.A., et al., "Sepsis and Septic Shock - A Review of Laboratory Models and a Proposal", *J. Surg. Res.* **29:** 189-201 (1980).
15. Fink, M.P., and S.O. Heard, "Laboratory Models of Sepsis and Septic Shock", *J. Surg. Res.* **49:** 186-196 (1990).
16. Olson, N.C., et al., "Systemic and Pulmonary Reactions of Swine to Endotoxemia and Gram-negative Bacteremia", *J. Am. Vet. Med. Assoc.* **200:** 1870-1884 (1992).
17. Olson, N.C., et al., "Endotoxemia and Gram-negative Bacteremia in Swine: Chemical Mediators and Therapeutic Considerations", *J. Am. Vet. Med. Assoc.* **200:** 1884-1893 (1992).
18. Olson, N.C., et al., "Biochemical, Physiological and Clinical Aspects of Endotoxemia", *Mol. Aspects. Med.* **10**: 511-629 (1988).
19. Dodds, W.J., "The Pig Model for Biomedical Research", *Fed. Proc.* **41:** 247-256 (1982).
20. Olson, N.C., et al., "Mediators and Vascular Effects in Response to Endotoxin", ***Br. Vet. J.*** **151:** 489-522 (1995).
21. Jesmok, G.J., et al., "Thromboxane-Blocked Swine as an Experimental Model of Severe Intravascular Inflammation and Septic Shock", *Shock* **4:** 379-383 (1995).
22. Bone RC, Balk RA, Cerra FB and the ACCP/SCCM Consensus Conference Committee: Definitions for sepsis and organ failure and guidelines for use of innovative therapies in sepsis Crit Care Med **20:** 864-874 (1992).

## Claims

1. Use of anti-TNF monoclonal antibodies for the manufacture of a pharmaceutical composition for treating septic shock in mammals wherein the shock is clinically established shock defined as having 2 or more of the following: fever, tachycardia, tachypnea, leukocytosis or leukopenia, the composition is administered in a solution at a dosage of about 1 to 10 mg of antibody per kilogram mammal body weight and the administration being within 1 - 12 hours after the onset of the established shock.
